# EUROPEAN PATENT SPECIFICATION

(11) **EP 4 096 429 B1**
(45) Date of publication and mention of the grant of the patent: **18.10.2023**
(21) Application number: 21702017.1
(22) Date of filing: 26.01.2021
(51) Int. Cl.: A23K 20/111, A23K 20/137, A23K 50/10, A61K 31/48

(54) **VETERINARY COMPOSITION FOR DAIRY RUMINANTS FOR TREATMENT OF MILK FEVER**
VETERINÄRMEDIZINISCHE ZUSAMMENSETZUNG ZUR BEHANDLUNG VON MILCHFIEBER BEI WIEDERKÄUERN FÜR DIE MILCHPRODUKTION
COMPOSITION VÉTÉRINAIRE POUR RUMINANTS LAITIERS POUR LE TRAITEMENT DE LA FIÈVRE DU LAIT

(30) Priority: 27.01.2020 EP 20305067
(43) Date of publication of application: 07.12.2022
(73) Proprietor: Ceva Santé Animale, 33500 Libourne (FR)
(72) Inventor: DEFLANDRE, Audrey, 33500 LIBOURNE (FR); ISAKA, Naomi, 33500 LIBOURNE (FR); BERTAIM, Thierry, 33500 LIBOURNE (FR)
(74) Representative: Cabinet Becker et Associés
(86) International application number: PCT/EP2021/051767
(87) International publication number: WO 2021/151898

(56) References cited:
- US-A1- 2011 245 261
- US-B2- 7 235 256
- P. LACASSE ET AL: "Review: Inhibition of prolactin as a management tool in dairy husbandry", ANIMAL, vol. 13, no. S1, 1 July 2019 (2019-07-01), pages s35-s41, XP055677643, GB ISSN: 1751-7311, DOI: 10.1017/S1751731118003312
- Anonymous: "Velactis: Pending EC decision | European Medicines Agency", , 13 September 2019 (2019-09-13), XP055677718, Retrieved from the Internet: URL:https://www.ema.europa.eu/en/medicines /veterinary/summaries-opinion/velactis [retrieved on 2020-03-19]
- Anonymous: "Cabergoline - DrugBank", , 13 May 2005 (2005-05-13), XP055678079, Retrieved from the Internet: URL:https://www.drugbank.ca/drugs/DB00248 [retrieved on 2020-03-19]
- HOP G E ET AL: "Efficacy of cabergoline in a double-blind randomized clinical trial on milk leakage reduction at drying-off and new intramammary infections across the dry period and postcalving", JOURNAL OF DAIRY SCIENCE, AMERICAN DAIRY SCIENCE ASSOCIATION, US, vol. 102, no. 12, 11 September 2019 (2019-09-11), pages 11670-11680, XP085904053, ISSN: 0022-0302, DOI: 10.3168/JDS.2019-16281 [retrieved on 2019-09-11]

## Description

The present invention relates to a veterinary composition and to a method for the treatment of post-partum hypocalcemia by administering said composition during dry period. The veterinary compositions and method of treatment according to the invention do not affect the milk-producing ability and/or milk quality of the ruminant during subsequent lactations.

In dairy ruminants, lactation generally lasts for 5 to 20 months. In dairy cows it is most often 10 months. After this lactation period, milking is stopped, generally suddenly, and the dairy ruminant is supposed to produce no more milk until calving, when the subsequent lactation begins. This period between the cessation of milking and the subsequent lactation, the latter being generally at parturition (or calving with respect to cows), known as the "dry period", is generally set at about 2 months (i.e. 56-70 days) in dairy cows.

Although a dry period is necessary to ensure optimal milk production during the subsequent lactation, this dry period necessitates several dietary changes and transitions to adapt to the physiological needs of an animal that is no longer producing milk. These dietary changes cause numerous physiological disruptions and stresses in ruminants, such as disruptions of the balance of the digestive flora, decreases in stomach capacity and absorption with changes in the size of the rumen and the villi of the ruminal mucosa. Finally, this dry period leads to a metabolism in ruminants that is very different from that observed during lactation. Thus, during the resumption of lactation after parturition, the ruminant is in a state of metabolic maladjustment resulting in an energy deficit, whereas the need to produce milk is again at a maximum.

With the initiation of lactation and continued milk production, tremendous adaptations occur in the dairy cow because of the increased need for nutrients to support milk synthesis. Besides the increased need for energy and amino acids for colostrum and afterward for milk synthesis, the requirement for calcium increases two- to three-fold over that required by the dairy cow before calving. Shortly before calving, a dairy cow deposits 8 to 10 g/d of calcium into her fetus, but when she calves, 20 to 30 g/d are secreted into colostrum and milk. Thus, metabolic adaptations must take place to support the increased need for calcium. If they do not take place soon enough or of sufficient magnitude, the concentration of calcium in the blood drops below a critical threshold and clinical and subclinical hypocalcemia, or milk fever, can result.

Calcium is vital for skeleton tissue and smooth muscle and nerve function including gastrointestinal motility and skeletal muscle strength. The lowest concentration of blood calcium usually occurs within 12 to 24 hours of calving and generally returns to normal in healthy cows within 2 to 3 days post-calving. Post-partum hypocalcemia, also known as Milk fever, is caused by a temporary blood calcium deficiency which usually occurs around the time of parturition and is one of the most common metabolic disorder in dairy cattle. Early clinical or preclinical symptoms (stage 1: the ruminant, and more specifically the cow, is still able to stand) can include excitability, nervousness, shifting of weight, and shuffling of hind feet. Post-partum hypocalcemia is a common cause of poor labor (dystocia), stillborn calves and apparent sudden death of dairy ruminants and more particularly dairy cows. Clinical hypocalcemia is the most recognized disease in dairy cattle by dairy farmers, with an incidence rate around 5%, which could sometimes reach 20%. Incidence increases with higher milk production and successive lactation.

The concentration of calcium in blood is tightly regulated through control of absorption of dietary calcium and release or uptake of calcium from bone. Two hormones, parathyroid hormone (known as PTH) and 1,25-dihydroxy vitamin D3, control these processes. As the concentration of calcium decreases in the blood, PTH is secreted and acts to decrease the excretion of calcium in the urine. This change allows for only small adjustments in the concentration of blood calcium. If greater amounts of calcium are needed, as with the initiation and maintenance of lactation, PTH acts on bone, and calcium is reabsorbed and released into the blood. In addition, PTH acts on the kidney and results in the conversion of a vitamin D metabolite into 1,25-dihydroxy vitamin D3. Then 1,25-dihydroxy vitamin D3 can regulate the absorption of calcium from the small intestine through active transport. In order for PTH to be secreted and effectively bind to its receptor, adequate magnesium and a slightly less alkaline blood pH (known as metabolic acidosis) are needed, thus illustrating the need to provide adequate amounts of magnesium in pre-fresh diets and balance these diets to provide a negative cation-to-anion difference (DCAD) in order to prevent hypocalcemia. However, such specific diets might not suffice to avoid hypocalcemia.

As specified earlier, some dairy cows may experience subclinical hypocalcemia which do not show clinical symptoms but have a low blood concentration of calcium usually within 24 hours after calving. A way to know whether dairy cows are experiencing subclinical hypocalcemia is to analyze blood for the concentration of calcium within the first 1 to 2 days after calving. Dairy cows with blood calcium concentrations at or below 8.8 mg/dl (2.2 mmol/l) but not showing clinical signs are considered subclinically hypocalcemic. It is estimated that the economic cost of subclinical hypocalcemia in a dairy herd is four times the cost of clinical cases, resulting in a substantial impact on profitability of dairy operations.

Post-partum hypocalcemia impacts fresh cow health, future milk production, and reproductive performance. Studies also have shown that immune function is compromised in dairy cows with low blood calcium concentrations.

Prevention of post-partum hypocalcemia generally occurs through modifications to the pre-fresh or close-up diet. These changes allow for the physiological system which mobilizes calcium to be primed and ready for the increased demand for calcium associated with the synthesis of colostrum and milk. However, such specific diets might be difficult to implement or might not suffice to avoid post-partum hypocalcemia.

There is a need for a method that prevents ruminants from post-partum hypocalcemia.

In veterinary medicine, prolactin inhibitor-based compositions such as Galastop^{®} (cabergoline, Ceva Santé Animale) can be administered as treatments for lactation during false pregnancies in bitches. They are also prescribed in order to stop milk production and suckling in ruminants. WO2010040765 in the name of the Applicant describes the administration of anti-prolactin dopamine receptor agonist compounds, such as cabergoline, to ruminants during gestation in order to induce lactation dry-off and to promote mammary involution, especially by intramuscular administration. However, such administration of cabergoline has never been implemented to treat or prevent post-partum hypocalcemia in ruminants. P. LACASSE ET AL: "Review: Inhibition of prolactin as a management tool in dairy husbandry", ANIMAL, vol. 13, no. S1, 1 July 2019 (2019-07-01), pages s35-s41, relates to prolactin inhibitors, such as cabergoline, and their use in dairy husbandry.

The Applicant has now discovered, surprisingly, that the administration of a veterinary composition comprising cabergoline to dairy ruminants during the dry period can treat post-partum hypocalcemia, and more specifically prevent from post-partum hypocalcemia.

### Summary of the invention

The present invention relates to veterinary composition comprising cabergoline for use to treat post-partum hypocalcemia of a dairy ruminant, wherein said composition is administered to the dairy ruminant during the dry period and preferably at the time of the implementation of the dry period, i.e. at the time of the dry-off.

According to the invention, said compositions are administered in effective therapeutic amounts, during the dry period and preferably at dry-off of dairy ruminants, in order to treat hypocalcemia in the subsequent lactation period(s), especially at and after parturition.

The compositions are administered to ruminants once and/or several times, preferably once.

The administration of veterinary compositions according to the invention does not affect the milk-producing ability and/or milk quality of the ruminant during the subsequent lactation.

The present invention also relates to kits for treating post-partum hypocalcemia in ruminants.

In one aspect there is provided a use of cabergoline, for manufacture of a veterinary composition for treating post-partum hypocalcemia in ruminants, wherein said composition is administered to ruminants during the dry period and preferably at dry-off of ruminants.

In another aspect there is provided a method for treating post-partum hypocalcemia in ruminants, wherein a veterinary composition comprising cabergoline is administered in a therapeutically effective amount during the dry period and preferably at dry-off of ruminants.

### Detailed description of the invention

The phrases "dry period" includes the period of time between last milking of the lactation period and the subsequent lactation of the subsequent lactation period, which starts generally at parturition. The dry period generally lasts about 2 months, more specifically from 45 to 70 days in dairy cows.

In the context of the invention, the terms "last milking", "dry-off' or "milking cessation" can be used interchangeably. They correspond to the time of the implementation of the dry period.

The terms "post-partum hypocalcemia" and "milk fever" can be used interchangeably and refer more specifically to clinical or subclinical hypocalcemia at parturition and more specifically at calving for dairy cows, and as detailed above. Dairy cows with blood calcium concentrations at or below 8.8 mg/dl (2.2 mmol/l) but not showing clinical signs are considered subclinically hypocalcemic. Clinical signs of milk fever of dairy ruminants include, but are not limited to tremor in muscles of the head and limbs, recumbency, lethargy, cold feeling of extremity, or hypothermia.

As used herein, the terms "treatment", "treating", and "treat" are defined as acting upon post-partum hypocalcemia with cabergoline or composition comprising the same to reduce or to anneal the risk of developing post-partum hypocalcemia or to reduce or alleviate one or more, preferably all, symptoms of post-partum hypocalcemia. The symptoms include, but are not limited to, tremor in muscles of the head and limbs, recumbency, lethargy, cold feeling of extremity, or hypothermia. It thus includes preventive and/or curative treatment. "Treatment," as used herein, includes: (a) reducing the risk of occurrence of post-partum hypocalcemia or milk fever in a dairy ruminant (b) impeding the initiation of reduction of blood calcium level, and/or (c) increasing blood calcium level or maintaining blood calcium level at a normal level, preferably to attain a blood calcium concentration higher than or equal to 2.1 mmol/l in ruminants or preferably to 2.2 mmol/l. The treatment, or more specifically prevention, of post-partum hypocalcemia includes the treatment, or more specifically prevention, of tremor in muscles of the head and limbs, recumbency, lethargy, cold feeling of extremity, or hypothermia due to hypocalcemia in dairy ruminants.

The present invention relates to a veterinary composition comprising cabergoline for use to treat, or more specifically to prevent from, milk fever, wherein said composition is administered to ruminants during the dry period and preferably at dry-off.

The present invention also relates to a veterinary composition comprising cabergoline administered in a single administration and/or in repeated administrations, preferably in a single administration, during the dry period and preferably at dry-off, for use to treating milk fever in dairy ruminants.

Administration of said compositions to dairy ruminants according to the invention allows more specifically to maintain a blood calcium concentration higher than or equal to 2.1 mmol/l in ruminants or preferably to 2.2 mmol/l. More particularly, said administration does not require any specific diet and more specifically any diet regimen aiming at maintaining a satisfactory blood calcium concentration in ruminants (such as diets with adequate amounts of magnesium and balance these diets to provide a DCAD). The compositions used according to the invention allow to maintain such blood concentration during the dry period and more specifically at parturition and after parturition, i.e. at the subsequent lactation.

The present invention also relates to a method of preventing and/or reducing post-partum hypocalcemia in dairy ruminants, as well as to a method of preventing dairy ruminants from milk fever, comprising an administration according to an effective therapeutic regimen of the veterinary compositions comprising cabergoline to ruminants during the dry period and preferably at dry-off.

Preferably, said veterinary compositions are administered to dairy cows.

More specifically, the veterinary compositions for use according to the invention are administered after the last milking. In a particular embodiment, the veterinary compositions are preferably administered within the first 5, the first 4 or the first 2 hours after the last milking, preferably within the first 30 minutes (such as 15-20 minutes) after the last milking.

The veterinary compositions and the methods according to the present invention are attractive to farmers as their use can maintain a satisfactory blood calcium concentration, without worrying about a specific blood calcium concentration increasing-regimen, resulting in a simplified herd management, and savings in terms of disease treatment costs when lactation is resumed. The administration of the compositions according to the present invention thus enables better management of dietary programs during the dry period.

Cabergoline, whose chemical name is N-[3-(dimethylamino)propyl]-N-[(ethylamino)carbonyl]-6-(2-propenyl)-8g-ergoline-8-carboxamide, is an anti-prolactin agonist compound specific for D2 dopamine receptors. It is in particular described in the patent US 4,526,892. Its chemical structural formula is the following:

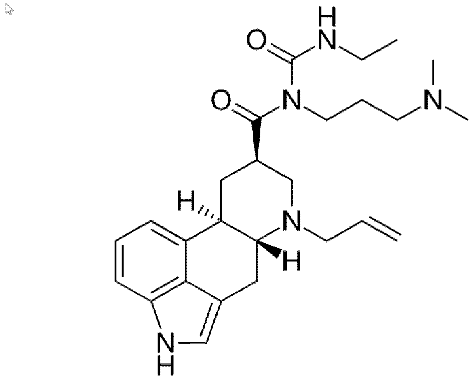

Cabergoline is the active ingredient in human drugs marketed under the names Dostinex^{®} and Cabaser^{®}. Also, it is the basic active ingredient in veterinary compositions marketed under the name Galastop^{®} for bitches prone to lactations of false pregnancy, and under the name Velactis^{®} for use in dairy cows for the reduction of udder involution duration during the dry period in the dairy cow and is administered as a single intramuscular injection. Neither of these cabergoline-based compositions, is administered to ruminants in order to treat milk fever in dairy ruminants.

The effective amounts or therapeutic doses are likely to vary according to the ruminants to be treated. The dosages, also called therapeutic regimens, can easily be determined by systematic tests on the basis of the example below and are within the ability of persons skilled in the art. According to a particular embodiment of the invention, the effective therapeutic doses according to the present invention are between 1 and 50 µg/kg, or between 5 to 11 µg/kg,and more preferably about 6 to 9 µg/kg.

More preferably, the amount of cabergoline which is administered is from 0.25 to 7 mg, preferably from 0.3 to 6 mg, and more specifically 1-6 mg, per dairy ruminant.

More preferably, the amount of cabergoline which is administered is from 2 to 7 mg, preferably from 3 to 6 mg, and more specifically 5-6 mg, per dairy cow.

According to the invention, the term "ruminants" refers to herbivorous mammals such as, for example, bovines, ovines, caprines, camelids or bovids. The dairy ruminants of the invention are preferably gestating ruminants. The compositions according to the invention are administered to milk-producing gestating ruminant mammals, preferably such as dairy cows, sheep and goats, and more preferably dairy cows.

Advantageously, the veterinary compositions of the present invention can be administered in association with standard treatments for treating and/or preventing intramammary inflammations and intra mammary infections (such as mastitis) of ruminants. Examples of standard care or prophylactic compositions of mastitis are local disinfectants for udders, antibiotics such as penicillins of group M, cephalosporine, gentamycin or even enzymes such as lysozymes or muramidase, and also teat-sealants

As mentioned before, post-partum hypocalcemia, also known as Milk fever, is caused by a temporary blood calcium deficiency which usually occurs around the time of parturition, more specifically at calving with respect to dairy cows. Milk fever generally occurs within the next 12-24 hours following parturition. According to a particular embodiment, post-partum hypocalcemia is treated within the next 12-24 hours following parturition.

According to a particular embodiment of the invention, the treatment of post-partum hypocalcemia of the invention includes reducing or annealing the risk of developing post-partum hypocalcemia, especially within the next 12-24 hours following parturition.

According to a particular embodiment of the invention, preventing and/or reducing post-partum hypocalcemia in a dairy ruminant refers to a treatment where the ruminant presents or attains a satisfactory blood calcium concentration which is generally higher than or equal to 2.1 mmol/l, preferably 2.2 mmol/L.

The veterinary compositions used according to the present invention can be administered according to many ways of administration that are well known in the field and adapted to the treatment of each of these animals. They are preferably administered orally or parenterally. It is even more preferred to administer them through intramuscular or subcutaneous injection or by intramammary administration. According to a particular embodiment, they are intramammary or preferably intramuscularly administered.

They can thus be in the form of an oral, intramammary or injectable liquid solution or suspension, or in solid or semi-solid form.

According to the formulation of the compositions used, they can further comprise ingredients conventionally used in pharmacy for the preparation of liquid formulations for intramammary administration.

Injectable preparations are prepared by mixing effective therapeutic amounts of cabergoline as described above with a solvent, a pH regulator, a buffer agent, a suspending agent, a solubilizing agent, a stabilizer, a tonicity agent and/or a preservative, and by transforming the mixture into a subcutaneous or intramuscular injection according to a conventional method.

As solvent, mention may be made of dimethylsulfoxide (DMSO), oily solvents such as medium-chain triglycerides, or a mixture of capric acid, caprylic acid and triglycerides such as that marketed under the name Mygliol^{®}812. As needed, the injectable preparations can be lyophilized according to a conventional method. Examples of suspending agents include methyl cellulose, polysorbate 80, hydroxyethylcellulose, xanthan gum, sodium carboxymethylcellulose and polyethoxylated sorbitan monolaurate. Examples of solubilizing agents include polyoxyethylene hardened castor oil, polysorbate 80, nicotinamide, polyethoxylated sorbitan monolaurate, macrogol and castor oil fatty acid ethyl ester. Furthermore, stabilizers include sodium sulfite, sodium metalsulfite and ether, while preservatives include methyl p-hydroxybenzoate, ethyl p-hydroxybenzoate, sorbic acid, benzyl alcohol, phenol, cresol and chlorocresol. An example of a tonicity agent is mannitol. During the preparation of the injectable solutions or suspensions, it is desirable to make sure that they are isotonic with blood.

The present invention further relates to a kit for veterinary use for treating, more specifically reducing or annealing, post-partum hypocalcemia, in dairy ruminants, where the kit includes a veterinary composition comprising cabergoline, as defined above, for use to treat post-partum hypocalcemia in dairy ruminants. The kits according to the present invention can include at least one compartment for an optionally sterile packaging including an effective therapeutic amount of cabergoline as described above, for administration to ruminants. The kit includes the means enabling the administration of the compositions by the chosen route, such as intramammary or intramuscularly route, as well as instructions relating to the mode of administration of the veterinary compositions according to the invention.

All embodiments described above for the composition and use of said composition also apply to the kit and to the method of the present invention.

Described herein below are examples. These examples are illustrative and in no way limiting.

### EXAMPLES

### Example 1: Cabergoline effect on milk fever in dairy cows

The cabergoline composition is a solution comprising cabergoline with dimethyl sulfoxide and medium-chain triglycerides, as excipients. It corresponds to the product called Velactis^{™} (cabergoline 1.12mg/mL).

The purpose of this clinical study was to assess the long-term safety of one intramuscular injection of cabergoline composition as detailed above after the last milking, at dry off, during the dry period and the subsequent lactation in dairy cows. It was a multicentric, parallel, randomized, blinded experimental design, evaluating the milk fever impact of the cabergoline composition as detailed above versus a negative control (Placebo) at calving after the dry period.

5.6 mg of the cabergoline composition were injected per dairy cow (i.e. 5ml/cow) for the Tested group. 5ml/cow of the vehicle of the cabergoline composition was injected to the control group (i.e.: 0 mg/ml of Cabergoline). Each animal was treated by intramuscular injection within 4 hours after the last milking at dry-off.

The total number of cows included in the study was 255 (126 in the Tested group and 129 in Placebo group). Both groups were comparable in their baseline criteria at inclusion.

Milk fever was assessed and recorded at calving in both groups by veterinarians.

The incidence of milk fever per group is given in Table 1.

**Table 1: Milk fever per group**

| Milk Fever detection | Tested Group (N=126) | Placebo group (N=129) | TOTAL (N=255) |
|---|---|---|---|
| No | 99.18%¹ (121)² | 89.52% (111) | 94.31% (232) |
| Yes | 0.82% (1) | 10.48% (13) | 5.69% (14) |
| Missing | (4) | (5) | (9) |

| | | | |
|---|---|---|---|
| ¹ percentage of cows presenting symptoms of Milk Fever ² number of cows into brackets | | | |

Considering all the calving criteria (abortion, premature calving, mode of calving, number and overall health status of calves, calving complications and pregnancy length) and all the breeding criteria (treatment for synchronisation of oestrus, first oestrus occurrence, pregnancy diagnosis, interval between calving and first oestrus, and between calving and fertilizing, ...) taken into account in the whole study, no relevant difference was observed between the Tested group and the Placebo group. In the same manner, the results of uterus and ovaries examination after calving for the cows treated with the cabergoline composition were comparable to those obtained in the animals treated with a Placebo and the milk production and Somatic Cell Count (SCC) recorded during all the lactation were similar between both groups. The milk production was even slightly higher in the Tested group compared to Placebo group.

Milk fever at calving (10.5% of the cows) was the most recorded in Placebo group. The percentage of cows with milk fever at calving was significantly lower in the Tested group compared to the Placebo group (p<0.05). It was also associated with a relative risk reduction in milk fever of around 92.2% with a CI95%= [41.2%; 99.0%] (calculated by statistical analyses).

In conclusion, a veterinary composition comprising cabergoline reduces the risk of developing milk fever in a very satisfactory and simple way. It is also important to note that such treatment does not affect the milk-producing ability and/or milk quality (e.g. SCC) of the ruminant during subsequent lactations.

## Claims

1. A veterinary composition comprising cabergoline for use to prevent a dairy ruminant from post-partum hypocalcemia, wherein said composition is administered to the dairy ruminant during the dry period, and preferably at dry-off.

2. The composition for use according to claim 1, to reduce or anneal the risk of developing milk fever in dairy ruminant,

3. The composition for use according to claim 1 or 2, wherein the composition is administered once and/or several times, preferably once.

4. The composition for use according to claim 1 or 2 or 3, wherein the composition is administered after the last milking.

5. The composition for use according to any one of the preceding claims, wherein the composition is administered within the first 5 or first 4 hours after the last milking, preferably within the first 30 minutes (such as 15-20 minutes) after the last milking.

6. The composition for use according to any one of the preceding claims, wherein the composition is administered at doses between 1 and 50 µg/kg,or between 5 to 11 µg/kg,and more preferably about 6 to 9 µg/kg.

7. The composition for use according to any one of the preceding claims wherein the administered amount of cabergoline is from 0.25 to 7 mg, preferably from 0.3 to 6 mg, and more specifically 1-6 mg, per dairy ruminant.

8. The composition for use according to any one of the preceding claims, wherein the administered amount of cabergoline is from 2 to 7 mg, preferably from 3 to 6 mg, and more specifically 5-6 mg, per dairy cow.

9. The composition for use according to any one of the preceding claims 1-6, wherein the ruminants are herbivorous mammals selected from bovines, ovines, caprines, camelids or bovids.

10. The composition for use according to any one of the preceding claims, wherein the ruminants are milk-producing ruminant mammals, preferably dairy cows, sheep or goats, and more preferably dairy cows.

11. The composition for use according to any one of the preceding claims, wherein the treatment reduces or anneals the risk of developing post-partum hypocalcemia, especially within the next 12-24 hours following parturition.

12. The composition for use according to any one of the preceding claims, wherein the composition is intramammary or preferably intramuscularly administered.

13. A kit including a veterinary composition such as defined in any one of the preceding claims, for use to treat post-partum hypocalcemia in dairy ruminants, wherein said composition is administered to the dairy ruminant during the dry period, and preferably at dry off.

## Patentansprüche

1. Veterinärmedizinische Zusammensetzung, umfassend Cabergolin zur Verwendung, um eine post-partum Hypokalzämie bei einem Milchvieh-Wiederkäuer zu verhindern, wobei die Zusammensetzung dem Milchvieh-Wiederkäuer während der Trockenzeit und vorzugsweise beim Trockenstellen verabreicht wird

2. Zusammensetzung zur Verwendung nach Anspruch 1, um das Risiko des Entwickelns von Milchfieber bei einem Milchvieh-Wiederkäuer zu reduzieren oder zu annullieren,

3. Zusammensetzung zur Verwendung nach Anspruch 1 oder 2, wobei die Zusammensetzung einmal und/oder mehrmals, vorzugsweise einmal, verabreicht wird.

4. Zusammensetzung zur Verwendung nach Anspruch 1 oder 2 oder 3, wobei die Zusammensetzung nach dem letzten Melken verabreicht wird.

5. Zusammensetzung zur Verwendung nach einem der vorstehenden Ansprüche, wobei die Zusammensetzung innerhalb der ersten 5 oder der ersten 4 Stunden nach dem letzten Melken, vorzugsweise innerhalb der ersten 30 Minuten (wie 15-20 Minuten) nach dem letzten Melken, verabreicht wird.

6. Zusammensetzung zur Verwendung nach einem der vorstehenden Ansprüche, wobei die Zusammensetzung in Dosen zwischen 1 und 50 µg/kg oder zwischen 5 bis 11 µg/kg und mehr bevorzugt etwa 6 bis 9 µg/kg verabreicht wird.

7. Zusammensetzung zur Verwendung nach einem der vorstehenden Ansprüche, wobei die verabreichte Menge an Cabergolin 0,25 bis 7 mg, vorzugsweise 0,3 bis 6 mg und insbesondere 1-6 mg pro Milchvieh-Wiederkäuer beträgt.

8. Zusammensetzung zur Verwendung nach einem der vorstehenden Ansprüche, wobei die verabreichte Menge an Cabergolin 2 bis 7 mg, vorzugsweise 3 bis 6 mg und insbesondere 5-6 mg pro Milchkuh beträgt.

9. Zusammensetzung zur Verwendung nach einem der vorstehenden Ansprüche 1 bis 6, wobei die Wiederkäuer pflanzenfressende Säugetiere sind, die aus Rindern, Schafen, Ziegen, Kamelen oder Hornträgern ausgewählt sind.

10. Zusammensetzung zur Verwendung nach einem der vorstehenden Ansprüche, wobei die Wiederkäuer milchproduzierende Wiederkäuer-Säugetiere, vorzugsweise Milchkühe, Schafe oder Ziegen, und mehr bevorzugt Milchkühe sind.

11. Zusammensetzung zur Verwendung nach einem der vorstehenden Ansprüche,
wobei die Behandlung das Risiko der Entwicklung von post-partum Hypokalzämie reduziert oder annulliert, insbesondere innerhalb der nächsten 12-24 Stunden nach der Entbindung.

12. Zusammensetzung zur Verwendung nach einem der vorstehenden Ansprüche, wobei die Zusammensetzung in die Milchdrüse oder vorzugsweise intramuskulär verabreicht wird.

13. Kit, einschließlich einer veterinärmedizinischen Zusammensetzung, wie in einem der vorstehenden Ansprüche definiert, zur Verwendung, um post-partum Hypokalzämie in Milchvieh-Wiederkäuern zu behandeln, wobei die Zusammensetzung dem Milchvieh-Wiederkäuer während der Trockenzeit und vorzugsweise beim Trockenstellen verabreicht wird.

## Revendications

1. Composition vétérinaire comprenant de la cabergoline pour utilisation en prévention d'une hypocalcémie post-partum chez un ruminant laitier, ladite composition étant administrée au ruminant laitier pendant la période de tarissement, et de préférence au tarissement.

2. Composition pour utilisation selon la revendication 1, pour réduire ou annuler le risque de développement de fièvre vitulaire chez un ruminant laitier,

3. Composition pour utilisation selon la revendication 1 ou 2, la composition étant administrée une fois et/ou plusieurs fois, de préférence une fois.

4. Composition pour utilisation selon la revendication 1 ou 2 ou 3, la composition étant administrée après la dernière traite.

5. Composition pour utilisation selon l'une quelconque des revendications précédentes, la composition étant administrée dans les 5 premières ou les 4 premières heures après la dernière traite, de préférence dans les 30 premières minutes (telles que 15 à 20 minutes) après la dernière traite.

6. Composition pour utilisation selon l'une quelconque des revendications précédentes, la composition étant administrée à des doses comprises entre 1 et 50 µg/kg, ou de 5 à 11 µg/kg, et plus préférablement d'environ 6 à 9 µg/kg.

7. Composition pour utilisation selon l'une quelconque des revendications précédentes dans laquelle la quantité administrée de cabergoline va de 0,25 à 7 mg, de préférence de 0,3 à 6 mg, et plus spécifiquement 1 à 6 mg, par ruminant laitier.

8. Composition pour utilisation selon l'une quelconque des revendications précédentes, dans laquelle la quantité administrée de cabergoline va de 2 à 7 mg, de préférence de 3 à 6 mg, et plus spécifiquement 5 à 6 mg, par ruminant.

9. Composition pour utilisation selon l'une quelconque des revendications 1 à 6 précédentes, dans laquelle les ruminants sont des mammifères herbivores choisis parmi bovins, ovins, caprins, camélidés ou bovidés.

10. Composition pour utilisation selon l'une quelconque des revendications précédentes, dans laquelle les ruminants sont des mammifères ruminants producteurs de lait, de préférence des vaches, moutons ou chèvres, laitiers, et plus particulièrement des vaches laitières.

11. Composition pour utilisation selon l'une quelconque des revendications précédentes,
dans laquelle le traitement réduit ou annule le risque de développement d'hypocalcémie post-partum, spécialement dans les 12 à 24 heures qui suivent la parturition.

12. Composition pour utilisation selon l'une quelconque des revendications précédentes, la composition étant administrée par voie intramammaire ou de préférence par voie intramusculaire.

13. Trousse comportant une composition vétérinaire telle que définie dans l'une quelconque des revendications précédentes, pour utilisation en vue de traiter une hypocalcémie post-partum chez des ruminants laitiers, ladite composition étant administrée au ruminant laitier pendant la période de tarissement, et de préférence au tarissement.
